# EUROPEAN PATENT SPECIFICATION

(11) **EP 1 176 962 B1**
(45) Date of publication and mention of the grant of the patent: **11.02.2004**
(21) Application number: 00926483.9
(22) Date of filing: 28.04.2000
(51) Int. Cl.: A61K 31/4709, A61K 31/496, A61K 31/535, A61K 9/127, A61P 31/04

(54) **LIPOSOME COMPOSITIONS FOR IMPROVED DRUG RETENTION**
LIPOSOMENZUSAMMENSETZUNGEN ZUR VERBESSERTEN WIRKSTOFFZURÜCKHALTUNG
COMPOSITION LIPOSOMIQUE POUR UNE RETENTION MEDICAMENTEUSE AMELIOREE

(30) Priority: 29.04.1999 US 131553 P
(43) Date of publication of application: 06.02.2002
(73) Proprietor: Alza Corporation, Mountain View, CA 94039-7210 (US)
(72) Inventor: GUO, Luke, S., S., Lafayette, CA 94549 (US); KIWAN, Radwan, Albany, CA 94706 (US)
(74) Representative: Williams, Paul Edwin
(86) International application number: PCT/US2000/011625
(87) International publication number: WO 2000/066126

(56) References cited:
- EP-A- 0 447 351
- WO-A-91/05545
- WO-A-96/37191
- WO-A-99/55743
- DATABASE WPI Section Ch, Week 198631 Derwent Publications Ltd., London, GB; Class A96, AN 1986-199682 XP002150310 & JP 61 130239 A (DAIICHI SEIYAKU CO), 18 June 1986 (1986-06-18)

## Description

### Field of the Invention

The present invention relates to a liposome composition which provides improved retention of a drug within the liposomes. In particular, the invention relates to liposomes including a fluoroquinolone antibiotic compound.

### Background of the Invention

Liposomes, or lipid vesicles, are a recognized drug delivery system which can improve the therapeutic activity and increase the safety of various pharmaceutical agents. For use as carriers *in vivo* of diagnostic or therapeutic compounds, the liposomes are typically prepared to contain the compound in liposome-entrapped from. To be useful in medical treatments, liposome formulations should have a high loading efficiency, *i.e*., a high drug to lipid ratio to reduce the non-therapeutic lipid load to the patient, and should have a practical shelf-life where the compound is entrapped with little leakage or loss of activity during storage.

There are two general method for preparing liposome formulations, passive loading, where drug and lipid are combined at the time of formation of lipid vesicles, and remote loading, where drug is loaded into the liposomes after lipid vesicle formation. In the passive loading method, a dried lipid film is typically hydrated with an aqueous phase medium containing the drug of interest, to form multi-lamellar vesicles which passively entrap the compound during liposome formation. The compound may be either a lipophilic compound included in the dried lipid film, or a water-soluble compound contained in the hydrating medium. For water-soluble compounds, this method gives rather poor encapsulation efficiencies, in which typically only 5-20% of the total compound in the final liposome suspension is in encapsulated form. Additional compound may be lost if the vesicles are further processed, *i.e.*, by extrusion, to produce smaller, more uniformly sized liposomes.

Other passive entrapment methods for forming compound-loaded liposomes are known, and solvent injection methods and a reverse-evaporation phase approach (Szoka, F.C., Jr., and Papahadjopoulos, D., *Proc. Natl. Acad. Sci. USA* 75:4194-4198 (1978)) have been proposed. These methods tend to suffer from relatively poor loading efficiencies and/or difficult solvent handling problems.

Remote loading of compounds has provided a means to overcomes some of the shortcomings of passive loading and in particular provides a methodology that can achieve a high drug to lipid ratio. In remote loading, an ion gradient, such as a hydrogen or ammonium ion gradient, is established across the liposome lipid bilayer and an ionizable compound is loaded into the liposomes.

However, there are recognized problems with remote loading against an ion gradient, one being that it is limited to drugs having an ionizable group. Moreover, not all ionizable drugs accumulate in the liposomes in response to an ion gradient (Chakrabarti, A., *et al*., U.S. Patent No. 5,380,532; Madden, T.D., *et al., Chemistry and Physics of Lipids* 53:37-46 (1990)). Another problem is that some agents which do accumulate in the liposomes are immediately released after accumulation. Yet another problem is that some agents which are successfully loaded and retained in the liposome *in vitro* have a high leakage rate from the liposomes *in vivo,* obviating the advantages of administering the agent in liposome-entrapped form.

### Summary of the Invention

Accordingly, it is an object of the invention to provide a liposome composition that includes an ionizable compound, in particular a fluoroquinolone, entrapped in the liposomes with superior retention.

It is another object of the invention to provide a method for preparing such a liposome composition.

In one aspect, the invention includes a liposome composition, composed of a suspension of liposomes formed of a vesicle-forming lipid. Contained within the liposomes is a fluoroquinolone compound and a polyanionic polymer effective to form a complex with the fluoroquinolone and to enhance retention of the fluoroquinolone in the liposomes.

In one embodiment, the fluoroquinolone is a 6-fluoroquinolone. In a preferred embodiment, the 6-fluoroquinolone is selected from the group consisting of norfloxacin, ciprofloxacin, ofloxacin, sparfloxcin, lomefloxacin, flerofloxacin, pefloxacin and amifloxacin.

In another embodiment, the polyanionic polymer is a polymer having anionic groups selected from sulfate, sulfonate, phosphate, and carboxylate groups. In particular, the polyanionic polymer is selected from dextran sulfate, chondroitin sulfate A, polyvinylsulfuric acid, and polyphosphoric acid.

The liposomes, in another embodiment, further include a lipid derivatized with a hydrophilic polymer, such as polyethylene glycol.

In a preferred embodiment, the liposomes include the polyanionic polymer dextran sulfate and the 6-flouorquinolone ciprofloxacin.

In another aspect, the invention includes a method for enhancing the retention of a 6-fluoroquinolone in a liposome. The method includes preparing liposomes composed of a vesicle-forming lipid and having an entrapped polyanionic polymer; and incubating the liposomes in the presence of a 6-fluoroquinolone compound under conditions effective to achieve uptake of the compound into the liposomes and complexing of the compound with the polyanionic polymer.

In yet another aspect, the invention includes a method of preparing a suspension of liposomes having a 6-fluoroquinolone entrapped in the liposomes in the form of a complex with a polyanionic polymer. The method includes the steps of forming a suspension of liposomes to have an internal aqueous phase including the polyanionic polymer and external phase including the polyanionic polymer; removing any unentrapped polyanionic polymer from the external phase by adding a salt effective to increase the ionic strength of the external bulk phase and which is effective to condense the polymer; loading a 6-fluoroquinolone into the liposomes by incubating the liposomes in the presence of the 6-fluoroquinolone under conditions effective to achieve uptake of the 6-fluoroquinolone into the internal aqueous phase of the liposomes; and removing any unentrapped 6-fluoroquinolone from the external phase.

In one embodiment, removing is by diafiltration of the condensed, polyanionic polymer.

The salt for the removing step is selected from the group consisting of NaCl, KCl, Na₂SO₄ and K₂SO₄.

These and other objects and features of the invention will be more fully appreciated when the following detailed description of the invention is read in conjunction with the accompanying drawings.

### Brief Description of the Drawings

Fig. 1A shows the general structure for quinolone antibiotic compounds;
Fig. 1B shows general structure for 6-fluoro-quinolones compounds;
Fig. 1C shows the structure of the 6-fluoro-quinolone ciprofloxacin;
Fig. 2 is a plot showing the amount of dextran sulfate in fractions collected from the permeate during diafiltration against water (open circles) and against 0.15 M NaCl (closed squares), 0.35 M NaCl (closed triangles), 0.45 M NaCl (closed circles) and 1.0 M NaCl (X symbols);
Fig. 3 is a plot showing the percentage of ciprofloxacin remaining in liposomes after incubation in plasma as a function of time for liposomes having a polyanionic polymer trapping agent (solid circles) and liposomes with no polymer trapping agent (open squares, open diamonds);
Fig. 4A is a plot showing the percentage of the injected dose of ciprofloxacin remaining in the bloodstream as a function of time after *in vivo* administration of liposomes containing dextran-sulfate-bound ciprofloxacin (closed circles) and of liposomes containing polyacrylic acid-bound ciprofloxacin (closed triangles) and of liposomes containing ciprofloxacin with no polymer trapping agent (open squares); and
Fig. 4B shows the concentration, in µg/mL, of ciprofloxacin in the bloodstream after intraveneous administration of liposome preparations of dextran sulfate/ciprofloxacin (closed circles), of polyacrylate-complexed ciprofloxacin (closed triangles) and of control liposomes having entrapped ciprofloxacin with no polymer trapping agent (open squares).

### Detailed Description of the Invention

### I. Liposome Composition

The present invention is concerned with providing a liposome composition including a quinolone antibiotic, and preferably a 6-fluoroquinolone, that is stably entrapped in the liposomes. This section describes the liposome components, including the entrapped drug and polyanionic polymer and the liposome lipid components. Preparation and characterization of the liposomes will be described in the sections below.

### A. Liposome-Entrapped Quinolone Compound

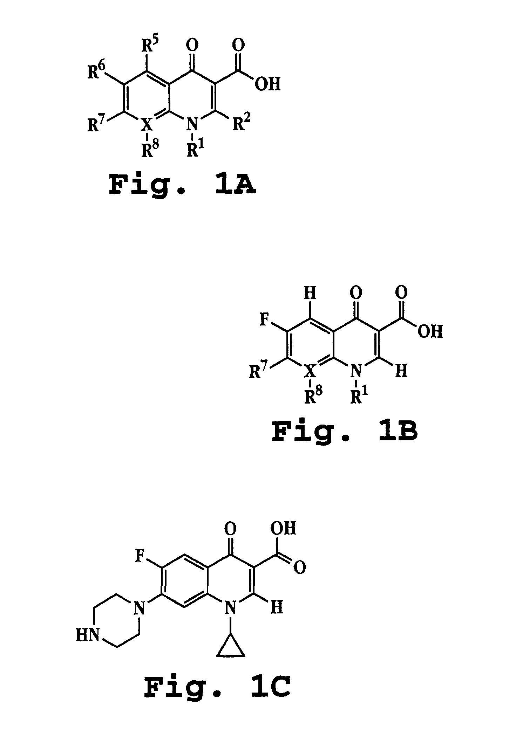

Quinolones are a class of synthetic antimicrobial agents of a general 4-pyridone-3-carboxylic acid structure, as shown in Fig. 1A. In the general structure shown in the figure, X is N or C and R₁, R₂, R₅, R₆, R₇ and R₈ can be any group. Some members of the quinolone class of drugs, such as nalidixic acid, have been available for many years. More recently, fluorinated-quinolones have been introduced, and these derivatives offer a therapeutic advantage over the earlier members in the class, as the fluorinated agents have broad antimicrobial activity and are effective after oral administration for the treatment of a wide variety of infections diseases. The compounds also have relatively few side effects and microbial resistance to their action does not develop rapidly. The general structure of the 6-fluoroquinolones is shown in Fig. 1B.

6-fluoro-quinolones have been widely studied and many structural variations are known, many of which are suitable for use in the present invention. One of the more potent 6-fluoro-quinolones is ciprofloxacin, (1-cyclopropyl-6-fluoro-1,4-dihydro-4-oxo-7-(1-piperazinyl)-3-quinoline carboxylic acid), the structure of which is shown in Fig. 1C. Like the other quinolones, ciprofloxacin acts by inhibiting bacterial DNA replication. The drug binds to the β-subunit of DNA gyrase, an essential enzyme for DNA replication that allows supercoils to be relaxed and reformed, and effectively inhibit its activity.

Ciprofloxacin includes a cyclopropyl ring at R1 and piperazine ring at R7, which includes an ionizable amine having a pKa of about 8.7. The drug is effective against most gram negative and some gram positive pathogenic bacteria. However, the therapeutic effectiveness of the antibacterial is limited by its short-elimination half-life of 3-4 hours, which makes it difficult to maintain a therapeutic concentration in the blood.

As described above, entrapment of drugs into liposomes is one approach to improving the therapeutic effectiveness of the drug, and entrapment of ciprofloxacin has been described (Ryan, J. *et al.*, WO 91/09616; Wong, J.P. *et al*., EP A1 0652008, Hope, M. *et al.,* WO 96/26715). However, liposomally-entrapped ciprofloxacin is not retained in the liposomes for a time sufficient to achieve the extent of *in vivo* biodistribution of long-circulating liposomes having a surface-coating of hydrophilic polymer chains. This is demonstrated in Comparative Example 1, which describes preparation of liposomes having a surface coating of polyethylene glycol chains and entrapment of ciprofloxacin in the liposomes by remote loading. The ciprofloxacin-containing liposomes were tested *in vitro* to measure the amount of ciprofloxacin that was released from the liposomes into plasma (using methodology as described in Example 5). It was found that almost all of the drug leaked from the liposomes after incubation in plasma for 4 hours at 37°C, with about 75% of the drug released within one hour.

Long-circulating liposomes remain in the blood stream for up to 24 hours. "Long-circulating" liposomes as used herein refers to liposomes having a surface coating of hydrophilic polymer chains, such as the polyethylene glycol-coated liposomes described in U.S. Patent No. 5,013,556. Up to 30% of the injected dose of long-circulating liposomes remains in the blood stream 24 hours after injection, in contrast to conventional liposomes, *e.g*., liposomes lacking the coating of polymer chains, which are cleared from the bloodstream in several hours. Thus, long-circulating liposomes achieve a biodistribution in the body that includes organs and tissues other than those of the mononuclear phagocyte system or reticuloendothelial system where conventional liposomes rapidly accumulate after administration (Paphadjopoulos, D. *et al., Proc. Natl. Acad. Sci. U.S.A.,* 88:11460-11464 (1991)). Clearly, a ciprofloxacin-liposome composition which releases more than half of the load of ciprofloxacin in a period of several hours due to leakage of the drug across the liposome bilayer does not take advantage of the long-circulating lifetime and good biodistribution of long-circulating liposomes.

Accordingly, a one aspect, the invention provides a liposome composition having ciprofloxacin entrapped within the liposomes and retained in the liposomes for a time sufficient to achieve biodistribution of the liposomes. In one embodiment, ciprofloxacin is entrapped in liposomes having a surface coating of hydrophilic polymer chains and the ciprofloxacin is retained in the liposomes for a time sufficient to take advantage of the long blood circulation lifetime of the liposomes, to achieve good biodistribution and sustained release of the entrapped compound.

More generally, the invention contemplates entrapping any number of compounds within the liposomes, an in particular any 6-fluoroquinolone. With reference again to Fig. 1B, 6-fluoro-quinolones are typically where R₁ is a C₁ to C₃ alkyl group, such as methyl, ethyl, propyl, or cyclopropyl, which may be substituted at one or more positions with F, Cl, or Br; R₇ is a linear, branched or cyclic nitrogen-containing alkane which preferably contains an ionizable amino group; exemplary R₇ substituents include ring structures such as 1-azetidyl, 1-piperazinyl, 4-methyl-1-piperazinyl, 4-ethyl-1-piperazinyl, 2-piperazinyl, 1-pyrrolidyl, each of which may be substituted with an NH₂, NHCH₃, N(CH₃)₂, 2-aminoethyl, ethylaminomethyl, 1-pyrrolidyl, or aminoethylaminomethyl group, other exemplary saturated and unsaturated nitrogen heterocycles include monocyclic heterocycles, *e.g.* imidazole, imidazoline, dihydropyrrole, 1,2,3,6-tetrahydropyridine, 1,4,5,6-tetrahydropyrimidine, pyrrolidine, morpholine, piperidine, piperazine, 1,3,5-triazine and bicyclic heterocycles, *e.g.* perhydroindole, perhydroquinoline, perhydroisoquinoline, perhydro-7-azaindole, perhydro-4-azabenzimidazole, 1,5-diazabicyclo[4.3.0]nonene (DBN), 1,8-diazabicyclo[5.5.0]undec-7-ene (DBU), 1,5-diazabicyclo[4.3.0]nonane, 1,8-diazabicyclo[5.5.0]undecane, 1,4-diazabicyclo[2.2.2]octane (triethylenediamine), 3-azabicyclo[3.2.2] nonane, 3-azabicyclo[4.3.0] nonane, 3-azabicyclo[3.3.0]octane, 2,8-diazabicyclo[4.3.0]nonane, 5-diazabicyclo[4.3.0]nonane, 1,5,7-triazabicyclo[4.4.0]dec-5-ene, 2,7-diazaspiro[4.4]nonane; X is N or C, such that when X is N, R₈ is nothing, and when X is C, R₈ is H, F, Cl, or CF₃, OCH₃, or OCH₂CH₃, or R₁ and R₈ together comprise a two- or three-atom alkyl or alkoxy bridge linking the 1 and 8 ring atoms in the quinolone ring to form a five- or six-member ring, respectively. Where cis- and trans- stereoisomers are possible, both isomers are contemplated. When asymmetric chiral centers are present, the compound may include a single stereoisomer or a mixture, typically a racemic mixture, of stereoisomers.

Some preferred 6-fluoroquinolones include ciprofloxacin, norfloxacin, ofloxacin, sparfloxacin, lomefloxacin, fleroxacin, pefloxacin and amifloxacin.

### B. Liposome Lipid Components

As discussed above, a quinolone compound is entrapped in liposomes, where "entrapped" refers to the compound being sequestered in the central aqueous compartment of the liposomes, in the aqueous space between liposome lipid bilayers, or within the bilayer itself. More specifically, in the present invention, the quinolone compound is entrapped in the liposomes in the form of a complex with the polyanionic polymer described above. "Complex" as used herein, refers to the species formed when the polymer and the quinolone are brought into contact. The complex can be a stable species in solution or a precipitate. In this section, liposomes for use in the invention are described.

The liposomes of the invention are composed of vesicle-forming lipids, generally including amphipathic lipids having both hydrophobic tail groups and polar head groups. A characteristic of a vesicle-forming lipid is its ability to form spontaneously into bilayer vesicles in water, as exemplified by the phospholipids. The vesicle-forming lipids of this type are preferably those having two hydrocarbon tails or chains, typically acyl groups, and a polar head group. Included in this class are the phospholipids, such as phosphatidylcholine (PC), phosphatidylethanolamine (PE), phosphatidic acid (PA), phosphatidylglycerol (PG), and phosphatidylinositol (PI), where the two hydrocarbon chains are typically between about 14-22 carbon atoms in length, and have varying degrees of unsaturation. Preferred phospholipids include PE and PC. An exemplary PC is hydrogenated soy phosphatidylcholine (HSPC). Single chain lipids, such as sphingomyelin (SM) may also be used.

The above-described lipids and phospholipids whose acyl chains have a variety of degrees of saturation can be obtained commercially, or prepared according to published methods. Other lipids that can be included in the invention are glycolipids. The term "glycolipid" as used herein encompasses lipids having two hydrocarbon chains, one of which is the hydrocarbon chain of sphingosine, and one or more sugar residues.

Lipids for use in the present invention may be relatively "fluid" lipids, meaning that the lipid phase has a relatively low lipid melting temperature, *e.g.*, at or below room temperature, or alternately, relatively "rigid" lipids, meaning that the lipid has a relatively high melting point, *e.g.*, at temperatures up to 50°C. As a general rule, the more rigid, *i.e.*, saturated lipids, contribute to greater membrane rigidity in the lipid bilayer structure, and thus to more stable drug retention after active drug loading. Preferred lipids of this type are those having phase transition temperatures above about 37°C.

The liposomes may additionally include lipids that can stabilize a vesicle or liposome composed predominantly of phospholipids. The most frequently employed lipid from this group is cholesterol at levels between 25 to 45 mole percent.

In one embodiment, the liposomes of the invention include a surface coating of a hydrophilic polymer chain. "Surface-coating" refers to the coating of any hydrophilic polymer on the surface of liposomes. The hydrophilic polymer is included in the liposome by including in the liposome composition one or more vesicle-forming lipids derivatized with a hydrophilic polymer chain. The vesicle-forming lipids which can be used are any of those described above for the first vesicle-forming lipid component, however, vesicle-forming lipids with diacyl chains, such as phospholipids, are preferred. One preferred phospholipid is phosphatidylethanolamine (PE), which contains a reactive amino group convenient for coupling to the activated polymers. One exemplary PE is distearyl PE (DSPE).

A preferred hydrophilic polymer for use in coupling to a vesicle forming lipid is polyethyleneglycol (PEG), preferably as a PEG chain having a molecular weight between 1,000-10,000 daltons, more preferably between 1,000-5,000 daltons.

Other hydrophilic polymers which may be suitable include polylactic acid, polyglycolic acid, polyvinylpyrrolidone, polymethyloxazoline, polyethyloxazoline, polyhydroxypropyl methacrylamide, polymethacrylamide, polydimethylacrylamide, and derivatized celluloses, such as hydroxymethylcellulose or hydroxyethylcellulose.

Preparation of lipid-polymer conjugates containing these polymers attached to a suitable lipid, such as PE, have been described, for example in U.S. Patent No. 5,395,619, and by Zalipsky in STEALTH LIPOSOMES (D. Lasic and F. Martin, Eds., CRC Press, Chapter 9 (1995)). Typically, between about 1-20 mole percent of the polymer-derivatized lipid is included in the liposome-forming components during liposome formation.

The hydrophilic polymer chains provide a surface coating of hydrophilic chains sufficient to extend the blood circulation time of the liposomes in the absence of such a coating. The extent of enhancement of blood circulation time is severalfold over that achieved in the absence of the polymer coating, as described in co-owned U.S. Patent No. 5,013,556.

Further, the liposomes may be prepared to contain surface groups, such as antibodies or antibody fragments, small effector molecules for interacting with cell-surface receptors, antigens, and other like compounds for achieving desired target-binding properties to specific cell populations. Here the lipid component included in the liposomes would include either a lipid derivatized with the targeting molecule, or a lipid having a polar-head chemical group that can be derivatized with the targeting molecule in preformed liposomes, according to known methods.

### C. Polyanionic Polymer

The liposomes in accordance with the invention further include a polyanionic polymer entrapped within the liposomes. The polymer is any molecule consisting of repetitive units of preferably similar chemical structure and having ionizable groups, that is, chemical functional groups capable of electrolytic dissociation resulting in the formation of ionic charge, and preferably an anionic charge. Polymers having a molecular weight over a broad range are contemplated, from 400-2,000,000 Daltons.

Polyanionic polymers suitable for use in the invention include sulfated, sulfonated, carboxylated or phosphated hydrophilic polymers. For example, sulfated proteoglycans, such as sulfated heparin, sulfated polysaccharids, such as sulfated cellulose or cellulose derivatives, carrageenin, dextran sulfate, mucin, sulfated polypeptides, such as polylysine with sulfated amine groups, glycopeptides with sulfonate-derivatized saccharide or peptide subunits, and hyaluronic acid. Chondroitin sulfates A, B and C, keratin sulfates, dermatan sulfates are also contemplated.

The polymer can also be a neutral polymer modified to include an anionic functional group. For example, amylose, pectin, amylopectin, celluloses, and dextran can be modified to include an anionic subunit.

Polymers bearing a sulfo group such as polyvinylsulfate, polyvinylsulfonate polystyrenesulfonate and sulfated rosin gum are also suitable.

As will be described below, the polyanionic polymer is entrapped in the liposomes during lipid vesicle formation. Upon loading of a drug into the liposomes, the polymer then serves to trap or retain the drug within the liposomes. In the studies conducted in support of the invention, dextran sulfate was used as an exemplary polyanionic polymer. Dextran sulfate is a polymer of anhydroglucose with approximately 2.3 sulfate groups per glucosoyl residue. It is composed of approximately 95% alpha-D-(1-6) linkages and the remaining (1-3) linkages account for the branching of dextran. The polymer is readily available in molecular weights ranging from 5,000 to 500,000 Daltons.

### II. Liposome Preparation

Liposomes for use in the present invention are prepared to have an inside to outside ion gradient across the lipid bilayer. The ion gradient is then used to load the desired compound into the liposomes. Liposomes having such an ion gradient, including hydrogen ion and ammonium ion gradient, are known to those of skill in the art and are readily prepared using known methodologies.

In the studies performed in support of the present invention, liposomes having an ammonium ion gradient across the lipid membrane were prepared. Preparation of such liposomes has been previously described, for example, in U.S. Patent No. 5,192,549. Here the liposomes are prepared in an aqueous buffer containing an ammonium salt, typically 0.1 to 0.4 M ammonium salt, such as ammonium sulfate or ammonium dextran sulfate, at a suitable pH, *e.g.*, 5.5 to 7.5. After liposome formation and sizing, the external medium is exchanged for one lacking ammonium ions, *e.g.*, the same buffer but one in which ammonium sulfate is replaced by NaCl or a sugar that gives the same osmolarity inside and outside of the liposomes.

After liposome formation, the ammonium ions inside the liposomes are in equilibrium with ammonia and protons. Ammonia is able to penetrate the liposome bilayer and escape from the liposome interior. Escape of ammonia continuously shifts the equilibrium within the liposome toward the right, to production of protons.

In accordance with another aspect of the invention, a method of preparing a suspension of liposomes having a quinolone antibiotic entrapped therein in the form of a complex with a polyanionic polymer is described. As described in Example 2, liposomes composed of hydrogenated soy phosphatidylcholine (HSPC), cholesterol and mPEG-DSPE (N-(carbonyl-methoxypolyethylene glycol 2000)-1,2-distearoyl-sn-glycero-3-phosphoethanolamine) were prepared. The lipids in a 50:45.2:4.8 molar ratio were dissolved in ethanol. The solubilized lipids were added to a solution of dextran sulfate ammonium salt, prepared as described in Example 2, and mixed for one hour to form multi-lamellar liposomes of heterogeneous size and having dextran sulfate ammonium salt entrapped within the liposomes.

The hydrated liposomes were extruded under pressure to size the liposomes to about 100-150 nm in diameter, as set forth in the example.

After liposome formation, the selected drug is loaded into the vesicles. The drug, however, will complex or precipitate in the presence of the polyanionic polymer. Thus any unentrapped polymer must be removed from the external suspension medium prior to drug loading to prevent undesirable complex formation in the liposome external medium. For the case of ciprofloxacin, precipitation occurs in the presence of even very small amounts of dextran sulfate, *e.g.*, 0.1 µg/mL. It is important to remove virtually all of the unentrapped polymer from the external medium.

According to an important feature of this aspect of the invention, the unentrapped polyanionic polymer is removed by diafiltration in the presence of a salt electrolyte. As described in Example 3, liposomes formed using dextran sulfate ammonium salt in the hydration medium were diafiltered against 350 mM NaCl using a hollow fiber ultrafiltration cartridge. The presence of the salt reduces the viscosity of the liposome suspension and condenses the polymer to facilitate removal of the dextran sulfate. Preferably, diafiltration is conducted under conditions which result in sufficient removal of any unentrapped polymer that no polymer/drug complex or precipitate is visible during the drug loading step.

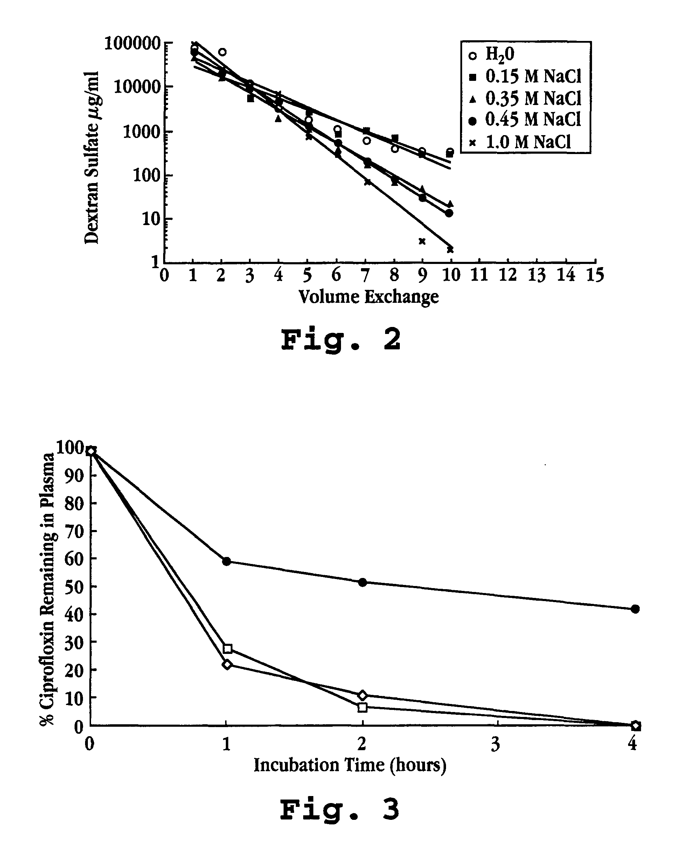

In studies conducted in support of the invention, removal of dextran sulfate sodium salt having an average molecular weight of 8,000 Daltons, was examined by diafiltration of the dextran sulfate against water and against various salt solutions differing in ionic strength. As described in Example 3, a dextran sulfate solution having a concentration of 100 mg/ml was diafiltered against 0.15 M NaCl, 0.35 M NaCl, 0.45 M NaCl and 1.0 M NaCl. The permeate from each diafiltration run was assayed for dextran sulfate and the results are shown in Fig. 2. As seen, dextran sulfate was more effectively removed when diafiltered against solutions having an increased ionic strength 0.15 M NaCl (closed squares), 0.35 M NaCl (closed triangles), 0.45 M NaCl (closed circles) and 1.0 M NaCl (X symbols)). Table 1 summarized the amount of dextran sulfate remaining in the retentate after 10 volume exchanges with the various salt solutions.

**Table 1**

| **Concentration of dextran sulfate remaining in the diafiltered solution after 10 volume exchanges** | |
|---|---|
| **NaCl Concentration (M)** | **Dextran sulfate remaining in retentate (µg/ml)** |
| 0 | 1088 |
| 0.15 | 1276 |
| 0.35 | 23 |
| 0.45 | 28 |
| 1.0 | 7 |

In general, it was found that greater than 99% of the dextran sulfate could be removed by increasing the ionic strength of the solution for diafiltration (*i.e*., from 0 mM to 350 mM NaCl) and adjusting the pH of the solution to a neutral range (pH=7.4).

It will be appreciated that a variety of electrolytes can be used to increase the ionic strength of the medium during diafiltration. Preferred are salts, such as NaCl, KCl, Na₂SO₄ and K₂SO₄. The amount of electrolyte for optimum removal of the polymer will, of course, vary with the polymer and the salt and the relative amounts of each. Generally, about 150 mM of an electrolyte is needed, with 450 mM being more preferable and up to 1000 mM possible. The upper limit of amount of electrolyte will in part be determined by stability of the liposomes in the solution, since an ionic gradient across the liposome bilayer is established and increases with increasing electrolyte concentration.

The pH of the diafiltration medium can also be adjusted to optimize removal of the polymer from the external medium. In the studies conducted in support of the invention, a neutral pH (*e.g.*, pH-7-7.5) gave good removal of dextran sulfate using NaCl as the electrolyte.

After removal of the unentrapped polymer, the selected drug is loaded into the liposomes by incubating a solution of the drug with the pre-formed liposomes under conditions effective to achieve loading of the drug into the liposomes. As described in Example 2, ciprofloxacin was loaded into the liposomes containing dextran sulfate by incubating an aqueous solution of the drug with the liposomes between 60-65°C for 30 minutes. Any unentrapped drug was then removed by diafiltration.

Table 2 summarizes the properties of four liposome preparations using the liposome preparation and diafiltration method described above. The preparations included as an exemplary quinolone ciprofloxacin, and the ciprofloxacin concentration was determined using spectrophotometry. The total lipid content was determined by measuring total phospholipid content. Particle size measurements were determined using dynamic light scattering.

**Table 2**

| **Liposomes with entrapped dextran-sulfate/ciprofloxacin** | | | | |
|---|---|---|---|---|
| **Batch No.** | **Ciprofloxacin Conc.** **(mg/mL)** | **total lipid conc.** **(mg/mL)** | **drug/lipid ratio** | **Particle Size** **(nm)** |
| 1 | 11.8 | 24.6 | 0.48 | 164 |
| 2 | 22.4 | 51.0 | 0.44 | 145 |
| 3 | 21.0 | 43.5 | 0.48 | 125 |
| 4 | 20.7 | 43.8 | 0.47 | 127 |

In other studies conducted in support of the invention, the polyanionic polymer polyacrylic acid was used as the trapping agent for ciprofloxacin. As described in Example 4, the lipids HSPC, cholesterol and mPEG-DSPE in a 40:45.2:4.8 molar ratio were hydrated with an aqueous solution of ammonium polyacrylate. After removal of the unentrapped polymer from the external medium via diafiltration in the presence of an electrolyte, the liposomes were loaded with ciprofloxacin. These liposomes, and the liposomes formed using dextran-sulfate as the trapping agent, were used for *in vitro* leakage tests and *in vivo* pharmacokinetic studies described below.

### III. Characterization of the Liposomes

Liposomes prepared as described above having ciprofloxacin entrapped in the form of a complex with dextran sulfate or with polyacrylate were characterized *in vitro* and *in vivo.*

More specifically, *in vitro* tests were conducted to determine the rate of leakage of ciprofloxacin from the liposomes in the presence of plasma. As described in Example 5, liposomes were prepared and incubated in plasma at a concentration of 50 µg ciprofloxacin/mL plasma. The liposomes were incubated in the plasma at 37°C for four hours, with samples taken at 0, 1, 2 and 4 hours for analysis.

The results are summarized in Tables 3A-3C in Example 5 and the percentage of ciprofloxacin remaining in the liposomes as a function of time is shown in Fig. 3. The liposomes prepared with the dextran sulfate trapping agent (closed circles) had a substantially lower leakage of ciprofloxacin, with 50% of the drug remaining in the liposomes after 2 hours of incubation. In contrast, the liposome preparations having entrapped ciprofloxacin with no polyanionic trapping agent (open square, open diamonds) had less than about 10% of the drug still in the liposomes after 2 hours of incubation in rat plasma.

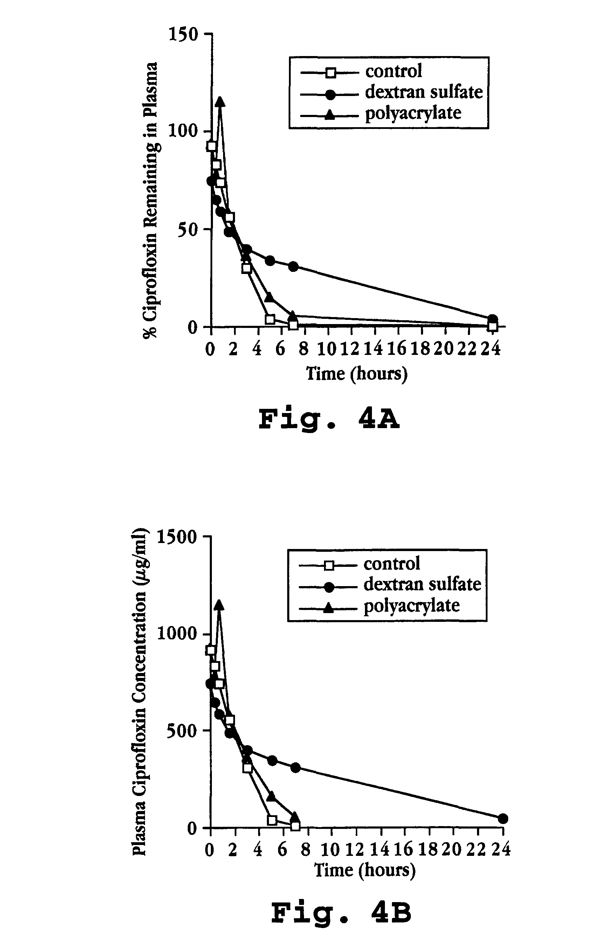

Liposome preparations containing dextran sulfate-entrapped ciprofloxacin and polyacrylate-entrapped ciprofloxacin were tested *in vivo* to determine the blood circulation kinetics of ciprofloxacin administered in liposome formulation prepared in accordance with the invention. As a control, liposomes containing ammonium sulfate/ciprofloxacin, that is with no polymer trapping agent, were also administered to animals. As described in Example 6, a single bolus dose (40 mg ciprofloxacin/kg) was administered to rats intravenously. Blood samples were collected from the animals at various time points up to 24 hours and analyzed for ciprofloxacin content. The results are summarized in Table 5 below and are shown graphically in Figs. 4A-4B.

Fig. 4A shows the results for liposomes containing dextran sulfate-bound ciprofloxacin (closed circles), polyacrylate-complexed ciprofloxacin (closed triangles) and for the control liposomes, *e.g.*, liposomes containing ammonium sulfate/ciprofloxacin with no polymer trapping agent (open squares). As seen, ciprofloxacin entrapped in liposomes with no polyanionic polymer (open squares) was rapidly released from the liposomes and cleared from the bloodstream, with about 50% of the injected dose removed about 1.5 hours after administration. In contrast, the liposomes having a polymer trapping agent exhibited significantly better retention of the ciprofloxacin, with 50% of the ciprofloxacin dose still present in the bloodstream after 2 hours for liposomes having dextran sulfate as the polymer trapping agent (solid circles) and polyacrylic acid as the trapping agent (solid triangles).

Fig. 4B is a similar plot showing the plasma concentration of ciprofloxacin in µg/ml for the test formulations, where liposomes with dextran sulfate-complexed ciprofloxacin (closed circles; see Table 5 for raw data); for liposomes having polyacrylate-complexed ciprofloxacin (closed triangles, see Table 5 for raw data) and for the control liposomes containing in the internal compartment ammonium sulfate-entrapped ciprofloxacin with no polymer trapping agent (open squares, see Table 5 for raw data). The improved blood circulation lifetime resulting from the enhanced retention of ciprofloxacin in the liposomes due to the trapping agent is evident. When administered in the form of liposomes with no polymer trapping agent, little ciprofloxacin remains in the bloodstream 24 hours after administration. In contrast, when the drug is administered in liposomes which include a polymer trapping agent, drug is still present at the 24 hour time point.

The pharmacokinetic parameters AUC (area under the curve) and Cmax for the dextran-sulfate bound ciprofloxacin, the polyacrylate-bound ciprofloxacin and the control formulation with no polymer trapping agent are summarized in Table 6.

**Table 6**

| **Pharmacokinetic Parameters for Liposomal Ciprofloxacin Formulations** | | |
|---|---|---|
| **Formulation** | **AUC** **(µg/ml x hour)** | **Cmax** **(µg/ml)** |
| dextran sulfate | 5772 | 754 |
| polyacrylate | 2452 | 925 |
| control | 2115 | 918 |

### IV. Examples

The following comparative example and examples illustrate preparation of liposomes including entrapped ciprofloxacin. The examples are in no way intended to limit the scope of the invention.

### Comparative Example 1

### Preparation of Liposomes Containing Ciprofloxacin

Liposomes having a surface-coating of polyethylene glycol were prepared by dissolving hydrogenated soy phosphatidylcholine (HSPC), cholesterol and polyethylene glycol derivatized to distearoyl phosphatidylethanolamine (PEG-DSPE) in a molar ratio of 50:45:5 respectively, in 50 ml ethanol at 60-65°C. A 350 mM solution of ammonium sulfate (pH 5.5) was prepared and heated to 60-65°C. The dissolved lipids were added to the ammonium sulfate solution and let mix for 1 hour at 60-65°C. The multilamellar liposomes obtained were extruded through 0.4µm, 0.2µm, 0.1µm, and 0.08 µm pore size polycarbonate membrane, 8 times each. The extrusion process was carried out at 60-65°C. Extruded liposomes had a mean diameter of approximately 100 nm.

The liposomes were then diafiltered against 350 mM ammonium sulfate (10 volume exchanges), then 5 mM NaCl containing 10% sucrose (10 volume exchanges).

A stock solution of ciprofloxacin was prepared at 58 mg/ml in 10% sucrose solution. Active drug loading was carried out by mixing the liposomes prepared as described above with the ciprofloxacin solution at a drug / lipid ratio of 0.71 mg/mg, and incubated at 60-65°C for 30 minutes, at which time an aliquot sample was taken and the percent of drug loading was determined using a Sephadex G-50 column. The percent of drug loading was 56% (loading efficiency up to 94% could be achieved using lower drug / lipid ratio).

The unencapsulated drug was removed by diafiltration against 10 mM histidine buffer pH 6.5, containing 10% sucrose. The ciprofloxacin-loaded liposomes were sterile filtered through 0.2 µm membrane and stored at 2-8°C. The final liposomes had a mean diameter of 101 nm and a drug / lipid ratio of 0.41 mg/mg.

Another preparation of liposomes containing ciprofloxacin having a mean diameter of 142 nm was produced using the same procedure described above with a slightly modified extrusion technique.

### EXAMPLE 2

### Preparation of Liposomes Containing Dextran Sulfate-ciprofloxacin

### A. Preparation of Dextran Sulfate Ammonium Salt

Dextran sulfate, sodium salt, molecular weight 8,000 Daltons (Dextran Product Limited (Ontario, Canada)) was converted to dextran sulfate ammonium salt as follows. An ion exchange column having a bed volume of 400 mL was packed with DOWEX 50X8-100 ion exchange resin (Aldrich Chemical Co, Milwaukee, WI). The packed column was rinsed with 2 L of 1 N HCl at a flow rate of 3-4 mUmin until the pH of the elute was approximately 1-2. The excess HCl was washed from the column with 2-3 L of deionized water until the pH of the eluting water was around 5.5.

300 mL of dextran sulfate sodium salt (50 mg/mL in water) was applied to the resin and collected in 12 50 mL fractions. The fractions containing dextran sulfate (fractions 3-12) were pooled and the pH adjusted from 1.29 to 5.5 with 5M ammonium hydroxide (approximately 25 mL).

The dextran sulfate ammonium salt was lyophilized in a 1 L round bottom flask in four 150 mL fractions. The lyophilized powder was dissolved in 5 mM NaCl at a concentration of 100 mg/mL. The solution pH was 5.4.

### B. Liposome Preparation

A solution of 8.75 mM dextran sulfate was prepared as described above and heated to between 60-65°C.

Hydrogenated soy phosphatidyl choline (HSPC, Lipoid K.G. Ludwigshafen, Germany), cholesterol (Croda, Inc. New York, NY) and mPEG-DSPE (Sygena, Ltd, Liestal Switzerland) were dissolved in a 50:45.2:4.8 molar ratio in dehydrated ethanol (Quantum Chemical, Cincinnati, Ohio) at 60-65°C. The solubilized lipids were added to the heated dextran-sulfate solution and the mixture was mixed at 60-65°C for one hour. The total lipid concentration was 106.38 mg/mL, or 150 µmol/mL.

The hydrated liposomes were extruded under pressure through a 0.2 µm pore size polycarbonate membrane filter at 60-65°C for 8-10 passes.

Unentrapped polymer was removed from the sized, liposome suspension by diafiltration against a NaCl solution. Using a hollow fiber ultrafiltration cartridge (NMCO 300,000; A/G Tech Corp. Needham, MA) the liposomes were diafiltered against 350 mM NaCl, pH 7.4 for 10 volume exchanges and then against 150 mM NaCl, pH 7.4, for 10 volume exchanges.

Sufficient ciprofloxacin (Bayer AG, Leverkusen, Germany) was dissolved in 150 mM NaCl to form a 30 mg/mL solution. The solution was heated to 60°C for complete dissolution. The drug solution was mixed with the liposome suspension and incubated at 60-65°C for 30 minutes, followed by rapid cooling to room temperature using an ice bath. The percent encapsulation of drug was measured by separation free drug from liposome vesicle on a size exclusion column and quantifying the amount of drug in each fraction by UV spectrophotometry.

Unentrapped drug was removed by diafiltration against 150 mM NaCl solution for 5 volume exchanges and then against a 10-volume exchange of 10% sucrose, 10 mM histidine, pH 6.5.

### EXAMPLE 3

### Diafiltration of Dextran-Sulfate Against Salt Solutions

Dextran sulfate sodium salt (molecular weight 8,000 Daltons, Dextran Products Limited, Scarborough, Ontario, Canada) was dissolved in distilled water at a concentration of 100 mg/ml. Diafiltration cartridge having a 300,000 molecular weight cut-off and 0.79 ft² surface area (A/G/ Technology Corporation, Needham, MA) was washed with 2 liters of distilled water. 90 mL of dextran sulfate were diafiltered against 1000 mL (about 10 volume exchanges) of distilled water. Volume exchanges (100 mL each) were collected separately, and 1 mL of each was used for dextran sulfate quantitation. The final retentate was brought up to the same original volume (90 mL) with water and then assayed for dextran sulfate content.

The same diafiltration procedure was repeated using 0.15 M NaCl, 0.35 M NaCl, 0.45 M NaCl and 1.0 M NaCl. Permeates were collected and analyzed for dextran sulfate using 1,9-dimethylene blue and absorbance at 590 nm. The results are shown in Fig. 2 and in Table 1.

### EXAMPLE 4

### Liposome Preparation with Polyacrylic Acid

An aqueous solution of 250 mM polyacrylic acid having a molecular weight of 2,000 Daltons (Aldrich Chemicals, Milwaukee, WI) and 50 mM ammonium sulfate was prepared and the pH of the solution was adjusted to 5.5 with 5 M NaOH.

Liposomes were prepared as described in Example 2 by hydrating the solubilized HSPC, cholesterol and mPEG-DSPE in a 50:45.2:4.8 molar ratio with the aqueous ammonium polyacrylate/ammonium sulfate solution. The unentrapped polymer was removed and ciprofloxacin was loaded into the liposomes according to Example 2.

### EXAMPLE 5

### In vitro Characterization

The rate of ciprofloxacin leakage from liposomes prepared according to Examples 2 and 3 was determined by incubating the liposomes at a concentration of 50 µg ciprofloxacin/mL plasma. The liposomes were incubated in the plasma at 37°C for four hours, with samples taken at 0, 1, 2 and 4 hours for analysis. Liposomes in each sample were separated from free drug by a solid phase extraction method (set forth below in Example 5B) and analyzed for ciprofloxacin content by reverse phase HPLC (set forth in Example 5C below). The results are summarized in Tables 3A-3C below and are shown in Fig. 3.

**Table 3A**

| **Liposomes with Ammonium Dextran Sulfate/Ciprofloxacin** | | | | |
|---|---|---|---|---|
| **Time** **(hours)** | **Liposomal Ciprofloxacin** **(µg/ml)** | **Free Cipro** **(µg/ml)** | **Total Cipro** **(µg/ml)** | **% cipro in liposomes** |
| 0 | 53.03 | 0.6 | 53.63 | 98.9 |
| 1 | 31.16 | 22.02 | 53.18 | 58.6 |
| 2 | 27.07 | 25.66 | 52.73 | 51.3 |
| 4 | 21.96 | 30.7 | 52.66 | 41.7 |

**Table 3B**

| **Liposomes with Ammonium sulfate/Ciprofloxacin** | | | | |
|---|---|---|---|---|
| **Time** **(hours)** | **Liposomal Ciprofloxacin** **(µg/ml)** | **Free Cipro** **(µg/ml)** | **Total Cipro** **(µg/ml)** | **% cipro in liposomes** |
| 0 | 48.52 | 0.99 | 49.51 | 98.0 |
| 1 | 13.44 | 35.01 | 48.45 | 27.7 |
| 2 | 3.18 | 44.96 | 48.14 | 6.6 |
| 4 | BQL* | 47.76 | BQL* | 0 |
| *BQL = below quantitation limit | | | | |

**Table 3C**

| **Liposomes with Ammonium Sulfate/Ciprofloxacin** | | | | |
|---|---|---|---|---|
| **Time** **(hours )** | **Liposomal Ciprofloxacin** **(µg/ml)** | **Free Cipro** **(µg/ml)** | **Total Cipro** **(µg/ml)** | **% cipro in liposomes** |
| 0 | 50.95 | 1.11 | 52.06 | 97.9 |
| 1 | 10.68 | 36.21 | 46.89 | 22.8 |
| 2 | 5.32 | 42.42 | 47.74 | 11.1 |
| 4 | BQL* | 45.89 | BQL* | 0 |
| *BQL = below quantitation limit | | | | |

### A. Solid Phase Extraction Method

### 1. Sample Preparation

The liposomal-ciprofloxacin formulation was diluted to a ciprofloxacin concentration of approximately 2 mg/mL in 10% sucrose/10mM histidine, pH 6.5. For each liposomal-ciprofloxacin formulation, 1.95 mL of blank rat plasma and was allowed to chill on ice. At time zero, 50 µL of chilled liposome formulation was mixed with the chilled plasma (final ciprofloxacin concentration of 50 µg/mL) and two 100µL aliquots were removed for immediate solid phase extraction for the zero time point. The plasma/liposome mixtures were then placed in a 37°C water bath. At 1, 2, and 4 hours, the mixtures were mixed and duplicate 100 µL aliquots were removed for extraction. A plasma blank was prepared in a similar manner, replacing the liposomal ciprofloxacin formulation with 10% sucrose/10mM histidine, pH 6.5 buffer. At 4 hours, an additional duplicate set of 100 µL aliquots from each plasma/liposome mixture was removed for total ciprofloxacin determination.

### 2. Solid Phase Extraction Method:

A SPE-24G solid phase extraction glass column processor (JT Baker, part #JT 7208-8) and Bond Elute LRC, C₁₈ cartridges for solid phase extraction, 100mg, 10mL (Varian part # 1211-3001) were used for the extraction procedure. 20 gauge disposable needles were attached below the cartridges to aid in sample collection. The cartridges were prewashed using 2mL methanol, followed by 2 mL saline. A moderate drip flow through the cartridges was achieved by using vacuum at about 4-6 in Hg and the C₁₈ packing bed was never allowed to dry until the end of the extraction procedure. Cartridges and needles were discarded after one use.

A 2mL volumetric flask was placed under the pre-washed C₁₈ cartridge for sample collection. The cartridge was then pre-treated with 200µL blank rat plasma to remove active sites on the C₁₈ sorbent. The 100 µL aliquot of plasma/liposome mixture was then loaded onto the C₁₈ bed. The cartridge was washed with 1 mL saline, collecting the eluent in the same flask below. This saline fraction contains the liposomal-ciprofloxacin and was labeled "S-Fraction".

The free ciprofloxacin was then washed from the C₁₈ cartridge with 1.6 mL methanol/0.1M NaH₂PO₄, pH 2.2 (9:1, v/v) (acidified methanol). The wash was collected in a second 2 mL volumetric flask and the C₁₈ packing was sucked dry, removing all of the sample. This fraction was labeled "F-Fraction".

The F-Fraction and S-Fraction were brought to 2.0 mL volume using saline. The 100 µL aliquots of the plasma/liposomal-ciprofloxacin mixture collected at 4 hours for determination of total ciprofloxacin was also diluted to 2.0 mL using saline and labeled "Total".

### B. HPLC Analysis for Ciprofloxacin:

### 1. HPLC Conditions:

Column temperature: 30°C
Flow: 1mL/min
Run time: 12 min.
Detection: UV at 278 nm
Mobile Phase: 25 mM NaH₂PO₄, pH 2.3/acetonitrile (87:13)
Injection volume: 20 µL
Column: Water Symmetry C₁₈ 4.6 x 150 mm, 5 micron, with precolumn.

The "Total" and "S-Fraction" were treated as follows. 250µL of the fraction was vortex mixed with 1000 µL acidified methanol then centrifuged at 3000 rpm (Sorvall RT6000) for 30 minutes, 10°C. The supernatants were transferred to HPLC vials for analysis.

The ciprofloxacin system precision sample and calibration standards were prepared in 15% blank rat plasma in saline and aliquots were treated with acidified methanol in the same manner as the "Total" and "S-Fraction". Ciprofloxacin and liposome entrapped-ciprofloxacin quality control samples were prepared in blank rat plasma, and 100 µL aliquots of the samples were diluted to 2.0 mL saline. Aliquots of the diluted samples were then treated with acidified methanol in the same manner as the "Total" and "S-Fraction". The HPLC calibration curve range was 0.02-10 µmg/mL and the results were fitted using least squares linear regression weighted by 1/(concentration)².

### EXAMPLE 6

### In vivo Characterization

### A. Liposome Formulations

Liposomes having the following compositions were prepared according to the procedure set forth in Example 1, 2 and 4 where active loading of liposomes was achieved using an ammonium sulfate concentration gradient after the liposomes were prepared by the ethanol hydration method.

**Table 4**

| **Liposome formulations for *in vivo* testing** | | | |
|---|---|---|---|
| **Component** | **Control Liposomes** ^{**1**} | **Dextran Sulfate Interior** | **Polyacrylate Interior** |
| HSPC² (mole %) | 50.0 | 50.0 | 50.0 |
| cholesterol (mole %) | 45.0 | 45.0 | 45.0 |
| mPEG-DSPE³ (mole %) | 5.0 | 5.0 | 5.0 |
| total lipid (mg/mL) | 36.7 | 41.4 | 35.7 |
| ciprofloxacin content (mg/mL) | 10.3 | 5.84 | 5.39 |
| internal buffer | ammonium sulfate | Ammonium Dextran sulfate | ammonium polyacrylate |
| external buffer | 10% sucrose, 10 mM histidine, 5 mM NaCl, pH 6.5 | 10% sucrose, 10 mM histidine, 5 mM NaCl, pH 6.5 | 10% sucrose, 10 mM histidine, 5 mM NaCl, pH 6.5 |
| extrusion temp (°C) | 60 | 60 | 60 |
| particle size (nm) | 102 | 114 | 94 |

| | | | |
|---|---|---|---|
| ¹ammonium sulfate interior | | | |
| ²hydrogenated soy phosphatidylcholine | | | |
| ³methoxypolyethylene glycol distearoyl phosphatidyl choline | | | |

### B. Animals

Eighteen male Sprague-Dawley rats (220 to 250 gms) were obtained from the vendor (Simonsen Labs, Gilroy, Ca) and housed in conventional housing under a 12 h:12 h light:dark cycle with food (Lab Diet™ Laboratory Rodent Diet # 5001) and water *ad libitum*. Animals were held at least three days prior to study initiation to ensure their good health.

### C. Study Design

Rats were administered a single intravenous injection via a lateral tail vein of one of the three liposomal-ciprofloxacin formulations at 40 mg/kg. Blood samples were obtained from anesthetized animals (inhaled isoflurane) via a retro-orbital sinus by collecting whole blood into 7.5% EDTA anticoagulant at the following times: 30 seconds 20 minutes, 40 minutes, 1.5 h, 3 h, 5 h, 7 h and 24 hours post-dose. Blood samples were stored on ice until centrifugation at 750 x g for 20 min at room temperature to obtain the plasma fraction. Plasma was frozen at-4 C until assayed for total ciprofloxacin (free plus encapsulated) by reverse-phase HPLC.

### D. Data Evaluation

The pharmacokinetics were obtained in each rat using weighted nonlinear regression with maximum likelihood estimation using the ADAPT II, Release 4.0 Pharmacokinetic/Pharmacodynamic Systems Analysis Software (D'Argenio and Schmutizky, ADAP II Users Guide. Biomedical Simulations Resource, University of Southern California, Los Angeles, CA). The empiric variance model for the ciprofloxacin concentrations assumed that the residual ('error') standard deviations of observations (σ) were linearly related to the true values. Model discrimination was by Akaikes' Information Criterion (AIC) (Akaike, H., A Bayesian Extension of the Minimum AIC Procedure of Autoregressive Model Fitting, *Biometika,* 66:237-242 (1979)). The maximum ciprofloxacin concentrations (Cmax) were obtained by direct observation of the plasma concentrations and the area under the curve at steady-state (AUCss) from time zero to infinity was determined by numerical integration.

The plasma ciprofloxacin concentrations are summarized in Table 5 and are shown graphically in Figs. 4A-4B. The pharmacokinetic parameters from the *in vivo* studies are shown in Table 6 above.

**Table 5**

| **Plasma Ciprofloxacin Concentrations** | | | |
|---|---|---|---|
| **Time** **(hours)** | **Ciprofloxacin in Blood** **(µg/ml)** | | |
| | **Control** | **Dextran Sulfate** | **Polyacrylate** |
| 0.083 | 918 ± 109 | 754 ± 83.9 | 925 ± 82.7 |
| 0.333 | 831 ± 13.8 | 648 ± 11.1 | 773 ± 57.0 |
| 0.66 | 742 ± 31.2 | 587 ± 18.9 | 1150 71.6 |
| 1.5 | 558 ± 27.1 | 491 ± 25.9 | 575 ± 32.1 |
| 3 | 304 ± 37.4 | 396 ± 29.0 | 363 ± 51.6 |
| 5 | 38.6 ± 15.8 | 345±16.2 | 154±41.9 |
| 7 | 6.5 ± 1.4 | 306 ± 22.6 | 49 ± 27.1 |
| 24 | BQL | 42.6 ± 22.6 | BQL |
| *BQL = below quantitation limit | | | |

## Claims

1. A liposome composition, comprising a suspension of liposomes composed of a vesicle-forming lipid; contained within the liposomes, a fluoroquinolone compound and a polyanionic polymer effective to form a complex with the fluoroquinolone and to enhance retention of the fluoroquinolone in the liposomes.

2. A composition according to claim 1, wherein said fluoroquinolone is a 6-fluoroquinolone.

3. A composition according to claim 2, wherein said 6-fluoroquinolone is a selected from the group consisting of norfloxacin, ciprofloxacin, ofloxacin, sparfloxacin, lomefloxacin, flerofloxacin, perfloxacin and amifloxacin.

4. A composition according to claim 1, 2 or 3, wherein the polyanionic polymer is a polymer including anionic groups selected from sulfate, sulfonate, phosphate, and carboxylate groups.

5. A composition according to claim 4, wherein the polyanionic polymer is selected from dextransulfate, chondroitin sulfate A, polyvinylsulfuric acid, and polyphosphoric acid.

6. A composition according to any preceding claim, wherein the liposomes further include a lipid derivatized with a hydrophilic polymer.

7. A composition according to claim 6, wherein the hydrophilic polymer is polyethylene glycol.

8. A composition according to claim 7, wherein the polyanionic polymer is dextran sulfate and the 6-fluoroquinolone is ciprofloxacin.

9. A method for enhancing the retention of a fluoroquinolone in a liposome composition according to any preceding claim, the method comprising
preparing liposomes composed of the vesicle-forming lipid, the liposomes having the polyanionic polymer entrapped therein;
incubating the liposomes in the presence of the fluoroquinolone compound under conditions effective to achieve uptake of the compound into the liposomes and complexation of the compound with the polyanionic polymer.

10. A method of preparing a suspension of liposomes according to any one of claims 1 to 8 having fluoroquinolone entrapped in the liposomes in the form of a coprecipitate with the polyanionic polymer, comprising forming a suspension of liposomes to have an internal aqueous phase including the polyanionic polymer and external phase including the polyanionic polymer;
removing any unentrapped polyanionic polymer from the external phase by adding a salt effective to increase the ionic strength of the external bulk phase and which is effective to condense the polymer;
loading the fluoroquinolone into the liposomes by incubating the liposomes in the presence of the fluoroquinolone under conditions effective to achieve uptake of the fluoroquinolone into the internal aqueous phase of the liposomes; and
removing any unentrapped fluoroquinolone from the external phase.

11. A method according to claim 10, wherein said removing includes removing by diafiltration the condensed, polyanionic polymer.

12. A method according to claim 10 or 11, wherein the salt is selected from the group consisting of NaCl, KCl, Na₂SO₄ and K₂SO₄.

## Patentansprüche

1. Liposomenzusammensetzung, umfassend eine Liposomensuspension, die aus einem vesikelbildenden Lipid besteht; eine In den Liposomen enthaltene Fluorchinolonverbindung und ein polyanionisches Polymer, das die Bildung eines Komplexes mit dem Flurorchinolon und die Verbesserung der Retention des Fluorchinolons in den Liposomen bewirkt.

2. Zusammensetzung nach Anspruch 1, worin das Fluorchinolon ein 6-Fluorchinolon ist.

3. Zusammensetzung nach Anspruch 2, worin das 6-Fluorchinolon ausgewählt ist aus der Gruppe, die aus Norfloxazin, Ciprofloxazin, Ofloxazin, Sparfloxazin, Lornefloxacin, Flerofloxacin, Perfloxazin und Amifloxacin besteht.

4. Zusammensetzung nach den Ansprüchen 1, 2 oder 3, worin das polyanionische Polymer ein Polymer ist, das anlonische Gruppen enthält, die aus Sulfat-, Sulfonat-, Phosphat- und Carboxylatgruppen ausgewählt sind.

5. Zusammensetzung nach Anspruch 4, worin das polyanionische Polymer ausgewählt ist aus Dextransulfat, Chondroitinsulfat A, Polvinylschwefelsäure und Polyphosphorsäure.

6. Zusammensetzung nach einem der vorhergehenden Ansprüche, worin die Liposomen weiterhin ein Lipid enthalten, dass mit einem hydrophilen Polymer derivatisiert ist.

7. Zusammensetzung nach Anspruch 6, worin das hydrophile Polymer Polyethylenglycol ist.

8. Zusammensetzung nach Anspruch 7, worin das polyanionische Polymer ein Dextransulfat und das 6-Fluorchinolon Ciprofloxacin ist.

9. Verfahren zur Verbesserung der Retention eines Fluorchinolons in einer Liposomenzusammensetzung nach einem der vorherigen Ansprüche, wobei das Verfahren
die Herstellung der Liposomen, die aus dem vesikelbildenden Lipid bestehen und wobei das polyanionische Polymer den Liposomen eingeschlossen ist;
das Inkubieren der Liposomen in Gegenwart der Fluorchinolonverbindung unter Bedingungen, die die Aufnahme der Verbindung in die Liposomen und die Komplexierung der Verbindung mit dem polyanionische Polymer bewirken, umfasst.

10. Verfahren zur Herstellung einer Liposomensuspension nach einem der Ansprüche 1 bis 8 mit Fluorchinolon, das in Form einer Mitfällung mit dem polyanlonischen Polymer in den Liposomen eingeschlossen ist, umfassend die Herstellung einer Liposomensuspension, die eine innere wässrige Phase, die das polyanionische Polymer. enthält, und eine äußere Phase hat, die das polyanionische Polymer enthält;
das Abtrennen von nicht-eingeschlossenem polyanionischem Polymer aus der äußeren Phase durch Zugabe eines Salzes, das die Erhöhung der lonenstärke des Hauptteils der äußeren Phase und das Kondensieren des Polymers bewirkt;
das Einbringen des Fluorchinolons in die Liposomen durch Inkubleren der Liposomen in Gegenwart des Fluorchinolons unter Bedingungen, die die Aufnahme des Fluorchinolons in die Innere wässrige Phase der Liposomen bewirken; und
das Abtrennen des nicht-eingeschlossenen Fluorchinolons aus der äußeren Phase.

11. Verfahren nach Anspruch 10, worin das Abtrennen die Abtrennung des kondensierten polyanionischen Polymer durch Diafiltration umfasst.

12. Verfahren nach Anspruch 10 oder 11, worin das Salz ausgewählt ist aus der Gruppe, die aus NaCl, KCl, Na₂SO₄ und K₂SO₄ besteht.

## Revendications

1. Composition liposomique, comprenant une suspension de liposomes composée d'un lipide de formation de vésicules ; contenu à l'intérieur des liposomes, un composé de fluoroquinolone et un polymère polyanionique capable de former un complexe avec la fluoroquinolone et d'augmenter la rétention de la fluoroquinolone dans les liposomes.

2. Composition selon la revendication 1, dans laquelle ladite fluoroquinolone est une 6-fluoroquinolone.

3. Composition selon la revendication 2, dans laquelle ladite 6-fluoroquinolone est choisie dans le groupe constitué par la norfloxacine, la ciprofloxacine, l'ofloxacine, la sparfloxacine, la loméfloxacine, la flérofloxacine, la perfloxacine et l'amifloxacine.

4. Composition selon l'une des revendications 1, 2 ou 3, dans laquelle le polymère polyanionique est un polymère comprenant des groupes anioniques choisis parmi les groupes sulfate, sulfonate, phosphate et carboxylate.

5. Composition selon la revendication 4, dans laquelle le polymère polyanionique est choisi parmi le dextran sulfate, le chondroïtine-sulfate A, l'acide polyvinylsulfurique et l'acide polyphosphorique.

6. Composition selon l'une quelconque des revendications précédentes, dans laquelle les liposomes comprennent en outre un lipide transformé en un dérivé par un polymère hydrophile.

7. Composition selon la revendication 6, dans laquelle le polymère hydrophile est le polyéthylène glycol.

8. Composition selon la revendication 7, dans laquelle le polymère polyanionique est le dextran sulfate et la 6-fluoroquinolone est la ciprofloxacine.

9. Procédé pour augmenter la rétention d'une fluoroquinolone dans une composition liposomique telle que définie à l'une quelconque des revendications précédentes, le procédé comprenant les opérations consistant à :
- préparer des liposomes composés du lipide de formation de vésicules, les liposomes ayant le polymère polyanionique piégé dans ceux-ci ;
- faire incuber les liposomes en présence du composé de fluoroquinolone dans des conditions capables de réaliser l'absorption du composé dans les liposomes et la complexation du composé par le polymère polyanionique.

10. Procédé de préparation d'une suspension de liposomes telle que définie à l'une quelconque des revendications 1 à 8, ayant de la fluoroquinolone piégée dans les liposomes sous la forme d'un coprécipité avec le polymère polyanionique, comprenant les opérations consistant à :
- former une suspension de liposomes pour avoir une phase aqueuse interne comprenant le polymère polyanionique et une phase externe comprenant le polymère polyanionique ;
- éliminer de la phase externe tout polymère polyanionique non piégé par addition d'un sel capable d'augmenter la force ionique de la phase d'ensemble externe et qui est capable de condenser le polymère ;
- charger la fluoroquinolone dans les liposomes par incubation des liposomes en présence de la fluoroquinolone dans des conditions capables de parvenir à l'absorption de la fluoroquinolone dans la phase aqueuse interne des liposomes ; et
- retirer toute fluoroquinolone non piégée à partir de la phase externe.

11. Procédé selon la revendication 10, dans lequel ladite élimination comprend l'élimination par diafiltration du polymère polyanionique condensé.

12. Procédé selon l'une des revendications 10 ou 11, dans lequel le sel est choisi dans le groupe constitué par NaCl, KCl, Na₂SO₄ et K₂SO₄.
